# EUROPÄISCHE PATENTSCHRIFT

(11) **EP 1 423 425 B1**
(45) Veröffentlichungstag und Bekanntmachung des Hinweises auf die Patenterteilung: **01.12.2010**
(21) Anmeldenummer: 02758115.6
(22) Anmeldetag: 23.07.2002
(51) Int. Cl.: C07K 14/705

(54) **REZEPTOR, DESSEN VERWENDUNG SOWIE MAUSANTIKÖRPER**
RECEPTOR, THE USE THEREOF, AND MOUSE ANTIBODIES
RECEPTEUR, SON UTILISATION, ET ANTICORPS DE SOURIS

(30) Priorität: 24.07.2001 DE 10136009; 09.03.2002 DE 10210425
(43) Veröffentlichungstag der Anmeldung: 02.06.2004
(60) Teilanmeldung: 10012352.0
(73) Patentinhaber: Debiovision Inc., Montreal, Quebec H3A 1E7 (CA)
(72) Erfinder: Müller-Hermelink, Hans Konrad, 97082 Würzburg (DE); Vollmers, Heinz, 97078 Würzburg (DE); Hensel, Frank, 97070 Würzburg (DE)
(74) Vertreter: Steinecke, Peter
(86) Internationale Anmeldenummer: PCT/DE2002/002699
(87) Internationale Veröffentlichungsnummer: WO 2003/011907

(56) Entgegenhaltungen:
- HENSEL FRANK ET AL: "A new variant of cystein-rich FGF receptor (CFR-1) specifically expressed on tumor cells." PROCEEDINGS OF THE AMERICAN ASSOCIATION FOR CANCER RESEARCH ANNUAL, Bd. 41, März 2000 (2000-03), Seite 698 XP001154022 91st Annual Meeting of the American Association for Cancer Research.;San Francisco, California, USA; April 01-05, 2000, March, 2000 ISSN: 0197-016X
- MOURELATOS ZISSIMOS ET AL: "Cloning and sequence analysis of the human MG160, a fibroblast growth factor and E-selectin binding membrane sialoglycoprotein of the Golgi apparatus." DNA AND CELL BIOLOGY, Bd. 15, Nr. 12, 1996, Seiten 1121-1128, XP001154021 ISSN: 1044-5498
- HENSEL FRANK ET AL: "Mitogenic autoantibodies in Helicobacter pylori-associated stomach cancerogenesis." INTERNATIONAL JOURNAL OF CANCER, Bd. 81, Nr. 2, 12. April 1999 (1999-04-12), Seiten 229-235, XP002250371 ISSN: 0020-7136 in der Anmeldung erwähnt
- VOLLMERS H PETER ET AL: "Human monoclonal antibodies from stomach carcinoma patients react with Helicobacter pylori and stimulate stomach cancer cells in vitro." CANCER (PHILADELPHIA), Bd. 74, Nr. 5, 1994, Seiten 1525-1532, XP009015348 ISSN: 0008-543X in der Anmeldung erwähnt
- VOLLMERS H PETER ET AL: "Adjuvant therapy for gastric adenocarcinoma with the apoptosis-inducing human monoclonal antibody SC-1: First clinical and histopathological results." ONCOLOGY REPORTS, Bd. 5, Nr. 3, Mai 1998 (1998-05), Seiten 549-552, XP009015315 ISSN: 1021-335X in der Anmeldung erwähnt
- HENSEL FRANK ET AL: "A novel proliferation-associated variant of CFR-1 defined by a human monoclonal antibody." LABORATORY INVESTIGATION, Bd. 81, Nr. 8, August 2001 (2001-08), Seiten 1097-1108, XP002250372 ISSN: 0023-6837

## Beschreibung

Offenbart wird ein an der Oberfläche von stark proliferierenden Zellen insbesondere des Magenkarzinoms befindlicher Rezeptor, dessen Verwendung, sowie die Struktur eines spezifisch hieran bindenden Mausantikörpers. Die vorliegende Erfindung betrifft diesen, in Anspruch 1 gekennzeichneten Antikörper.

Weithin bekannt ist, für klinische und wissenschaftliche Untersuchungen von Hybridomen erzeugte monoklonale Antikörper einzusetzen. Für die Behandlung von Tumoren, viralen und mikrobiellen Infektionen, B-Zell-Immundefizenzen mit verringerter Antikörpererzeugung und andere Störungen des Immunsystems ist die Anwendung humaner monoklonaler, von B-Zell Hybridomen erzeugter Antikörper vielversprechend.

Beim Magenkarzinom handelt es sich um eine der weltweit häufigsten Krebsarten. Nach Lauren "The two histological man types of gastic carcinoma", Acta Path Microbiol Scand; 64:331-49, werden sie histologisch eingeteilt in diffuse Adenokarzinome und intestinale Adenokarzinome. Intestinale Magenkarzonome sind oft von chronischer Gastristis B begleitet und insbesondere von intestinalen Metaplasien, die als Vorläufer dysplastischer Veränderungen und von Magenkarzinomen betrachtet werden. Unterschiede zwischen diesen beiden Arten zeigen sich auch darin, dass Patienten mit Karzinomen des diffusen Typs oft der Blutgruppe A angehören, woraus auf den Einfluss genetischer Faktoren beim Krebsrisiko geschlossen werden kann, währen Umweltfaktoren, z.B. eine Helicobacter pylori-Infektion, möglicherweise für die Entstehung von Karzinomen des intestinalen Typs von Bedeutung sind. Zwar ist eine abnehmende Häufigkeit der Magenadenokarzinome im Westen festzustellen, dafür treten sie aber nun vermehrt im Osten auf.

Die Entwicklung von Magenkrebs ist ein mehrstufiger und multifaktorieller Prozeß (Correa, 1992 Cancer Res. 52:6735-6740). Obwohl nur wenig über die molekularen Mechanismen bekannt gelten als Faktoren hohe Salzeinnahmen, Alkohol, Nitrosamine und Infektionen mit den Bakterium Helicobacter Pylori (H. pylori) als nachweislich am Entstehen des Magenkrebses beteiligt. Aufgrund des engen Zusammenhangs zwischen H. pylori Infektionen und dem Vorkommen von Gastritis dysplasi und der Entwicklung von Magenkrebs wurde dieses Bakterium durch die WHO als in die Klasse 1 der kanzerogenen Stoffe gehörend klassifiziert. H. pylori induziert direkt schwerwiegende vorkanzerogene Änderungen der Zellen im Bereich der Schleimhaut und ist ebenfalls verantwortlich für ein Ansteigen der Autoantikörper, die häufig bei Gastritis und Magenkrebspatienten beobachtet werden (Negrini et al., 1996 Gastroenterol. 111:655-665). Diese Antikörper sind in der Lage, Magenlessionen und Apoptose im Magenepithel zu induzieren (Steiniger et al., 1998 Virchows Arch. 433:13-18). Die Natur der Antigene ist teilweise bis heute unbekannt. Antikörper gegen die Magen H+/K(+)-ATPase (Claeys et al., 1998 Gastroenterology 115:340-347), Interleukin-8 (Crabtree et al., 1993 Scand.J.Immunol. 37:65-70; Ma et al., 1994 Scand.J.Gastroenterol. 29:961-965) und das Lewisblutgruppenantigen (Appelmelk et al., 1997 Trends.Microbiol. 5:70-73) werden häufig bei Magenschleimhaut oder Magenkrebs gefunden.

Die Therapie war bislang auf Gastrektomie und Lymphadenektomie beschränkt, aufgrund der auch dann noch schlechten Prognose besteht jedoch der Bedarf nach einer neuen begleitenden Therapie. Immunologische Studien haben gezeigt, dass auch in Fällen, in denen das Immunsystem maligne Zellen nicht wirksam bekämpfen kann, eine zelluläre und humorale Aktivität meßbar ist, aber nicht ausreicht, um die Tumorzellen zu zerstören. Ein wirkungsvoller Ansatz ist nun der, von der Immunantwort des Patienten stammenden Antikörper zu isolieren, geeignet zu vermehren und therapeutisch einzusetzen. So wurden bsp. von Patienten mit Lungen-, Ösophagus- und Dickdarmkrebs stammende Antikörper isoliert und davon humane monoklonale Antikörper abgeleitet, die z.B. direkt Differenzierung und das Wachstum der Tumorzellen beeinflussen.

Apoptose ist der programmierte Zelltod, Selbstmord von Zellen, durch Fragmentation der DNA, Zellschrumpfung und Dilatation des endoplasmatischen Reticulums, gefolgt von Zellfragmentation und der Bildung von Membranvesikeln, den sog. apoptotischen Körpern. Apoptose, die physiologische Form des Zelltods, garantiert eine schnelle und saubere Entfernung unnötiger Zellen, ohne Entzündungsvorgänge oder Gewebeverletzungen auszulösen wie im Falle der Nekrose. Unter pathologischen Bedingungen dient sie auch zum Entfernen maligner Zellen, wie etwa Krebsvorläuferzellen. Sie kann durch verschiedenste Stimuli ausgelöst werden, wie etwa durch zytotoxische T-Lymphozyten oder Zytokine, wie Tumornekrosefakor, Glukokortikoide und Antikörper. Sie ist die häufigste Todesursache eukaryontischer Zellen und kommt vor in der Embryogenese, Metamorphose und Gewebsatrophie. Apoptotische Rezeptoren an der Zelloberfläche, wie jene der NGF/TNF-Familie werden prädominant auf Lymphozyten exprimiert, befinden sich aber auch auf verschiedenen anderen Zelltypen, weshalb sie sich nicht für eine Krebstherapie eignen. Insbesondere haben bei in-vivo-Tests Liganden und Antikörper für diese Rezeptoren zu Leberschäden geführt. Deshalb sind tumorspezifische Rezeptoren mit apoptotischer Funktion besonders wichtig.

In jüngsten Publikationen wurde der humane Antikörper 103/51 beschrieben, der von Magenkrebspatienten mit diffusem Adenokarzinom gewonnen wurde und der mit H. pylori und Magenkrebszellen wechselwirkt (Vollmers et al., 1994 Cancer 74:1525-1532). Bei allen Untersuchungen wurde die bekannte Magenadenokarzinom-Zellinie 23132 verwendet, die unter der Nr. ACC201 bei der DSMZ-Deutsche Sammlung von Mikroorganismen und Zellkulturen GmbH, Mascheroder Weg 1 b, 38124 Braunschweig hinterlegt ist. Der Antikörper hat in geringen Dosen einen mitotischen Effekt auf Magenkrebszellen in vitro, in dem sie an einen 130 kD Membranrezeptor binden (Hensel et al., 1999 Int.J.Cancer 81:229-235). Durch Sequenzierung der Genbereiche des Antikörpers wurde der Antikörper 103/51 als Autoantikörper identifiziert. Immunohistochemische Untersuchungen zeigen, daß der Antikörper stark mit Magenkrebszellen und mit Magenhormonzellen reagiert.

Der zelluläre Rezeptor des monoklonalen Antikörpers 103/51 war bisher nicht bekannt. Im Rahmen der zu vorliegenden Erfindung führenden Untersuchungen konnte dieser zelluläre Rezeptor identifiziert werden: Diese Identifizierung gestaltete sich jedoch als schwierig. Einerseits reagiert der monoklonale Antikörper 103/51 bei der Westernblot-Analyse mit seinem Rezeptor nur unter ganz bestimmten Stringenzbedingungen. Andererseits findet man eine durch Denaturierungsartefakte hervorgerufene unspezifische Reaktion mit einer Reihe weiterer Proteine.

Sequenzierungen haben gezeigt, daß der Rezeptor Übereinstimmungen mit dem CFR-1 Protein aufweist, jedoch mit diesem nicht identisch ist. Des weiteren werden damit Glycoprotein-Verbindungen beansprucht, die in einer oder mehreren Determinanten (Liganden) mit denen des bekannten CFR-1 übereinstimmen. Insbesondere wird eine Homologie gefordert, die im Sinne dieser Anmeldung als eine Übereinstimmung von mindestens 80% in den primären Aminosäuresequenzen zu definieren ist. Der Rezeptor ist demzufolge eine Isoform zu CFR-1. Zusätzlich ist eine spezifische Bindung entweder an den humanen Antikörper 103/51 und/oder den murinen Antikörper 58/47-69 gefordert.

Von besonderem Interesse ist, wenn die spezifische Bindungsstelle am Glykoprotein ein Carbohydrat-Rest, also ein Zucker-Rest ist.

In spezieller Ausgestaltung weist das CFR-1 Protein als Determinante eine Aminosäuresequenz gemäß Anlage S Zellinie 23132 auf.

Bei dem zellulären Rezeptor des Antikörpers 103/51 handelt es sich um eine für Tumorzellen, insbesondere für Magenkarzinomzellen spezifische Isoform des Proteins CFR-1, die in normalen Gewebe nicht auftritt. Die spezifischen Rezeptoreigenschaften dieser Isoform beruhen auf einer besonderen mit dem Proteinrückgrat über eine N-Verknüpfung verbundenen Glykostruktur. Die tumorspezifische Rezeptor kann in einem Screeningverfahren zur Identifizierung von spezifischen Bindepartnern eingesetzt werden. Spezifischer Bindepartner an den Rezeptor sind im Sinne der vorliegenden Erfindung solche Substanzen, die selektiv an eine tumorspezifische Glykostruktur von CFR-1 binden und vorzugsweise die Fähigkeit zur Apoptoseinduzierung besitzen. Diese spezifischen Bindepartner können für die Herstellung von therapeutischen Mitteln zur Tumorbekämpfung sowie zur Herstellung von diagnostischen Mitteln eingesetzt werden.

Die Proteinverbindung wurde als Isoform des CFR-1 durch Reinigung, Sequenzierung und Transfektion bestimmt. Die Spezifizität für das Antigen 103/51 wird durch Herstellung muriner Antikörper aus gereinigten Molekülen mit identischen Reaktionen und Funktionen bestätigt, durch immunhistochemisches Färben und einer MTT-Untersuchung von zwei CFR-1 negativen Zellinien. Die Isoform des CFR-1 Moleküls, welches sowohl durch den humanen als auch den murinen Antikörper festgestellt wurde, ist in den Zellmembranen der Epithelzellen lokalisiert und hat ein Expressionsmuster, das sich von den bisher für CFR-1 beschriebenen unterscheidet (Burrus et al., 1992 Mol.Cell.Biol. 12:5600-5609).

Das CFR-1, welches aus hochaffinen FGF Bindeprotein (FGF=fibrolast growth factor) aus Hühnerfibroplasten isoliert wurde (Burrus et al., 1992 Mol.Cell.Biol. 12:5600-5609), bindet in einer größeren Anzahl an FGFs und dürfte eine Rolle spielen bei der Regulierung der Zellproliferation. In Eizellen chinesischer Hamster (CHO) wurde CFR-1 im Golgi-Apparat (Burrus et al., 1992 Mol.Cell.Biol. 12:5600-5609) gefunden, aber es kann ebenso in einer mutierten Form ausgeschieden werden (Zuber et al. 1997 J.Cell Physiol. 170:217-227). In Abhängigkeit vom Organismus wurden zwei Varianten des CFR-1 festgestellt, die Homologien zwischen 80 und 95% mit ESL-1 (= E-selectin-ligand 1) und MG-160 (Membransialoglykoprotein 160) aufweisen (Burrus et al., 1992 Mol.Cell.Biol. 12:5600-5609; Stieber et al., 1995 Exp.Cell.Res. 219:562-570; Steegmaier et al., 1995 Nature 373:615-620; Mourelatos et al., 1996 DNA Cell.Biol. 15:1121-1128) und scheinen keine weiteren Sequenzhomologien mit anderen, soweit bekannten Proteinen aufzuweisen. Die Funktion und der zellulare Aufbau der CFR-1 und deren Homologen ist relativ unbekannt und widersprüchlich. Es konnte gezeigt werden, daß MG-160, welches ein mittleres Golgi sialoglycoprotein dargestellt und aus Rattenhirn gewonnen wurde, eine Rolle im intrazellulären FGF Austausch spielt (Zuber et al., 1997 J.Cell.Physiol. 170:217-227).

Jüngste Resultate haben gezeigt, daß die Lokalisierung dieses Proteins nicht auf den Golgi Apparat beschränkt ist. Dann jedoch, wenn es am c-terminus abgeschnitten ist, kann das Protein an der Plasmamembran und den Filopodia lokalisiert sein (Gonatas et al., 1998 J.Cell.Sci. 111:249-260). Dies stimmt überein mit der Kenntnis, daß das dritte Homologe, ESL-1, welches aus mausneutrophilen Progenitorzellen isoliert wurde (32Dcl3), sowohl im Golgi-Apparat als auch an der Zelloberfläche von microvilli gefunden wurde (Steegmaier et al., 1997 J.Cell.Sci. 110:687-694; Gonatas et al., 1998 J.Cell.Sci. 111:249-260). ESL-1 wurde als Ligand von E-selectin in neutrophilen Zellen mit einem ungefähren Molekulargewicht von 150 kD identifiziert. Immunpräzipitation mit Anti ESL-1 Antikörpern zeigen, daß eine nicht näher definierte Isoform dieses Proteins aus verschiedenen Zellen ausgefällt werden können, einschließlich einiger Krebszellinien (Steegmaier et al., 1995 Nature 373:615-620).

Aufgrund der überwiegend in den Membranen von Krebszellen liegenden Vorkommen des CFR-1, ist der Schluß zu ziehen, daß der beschriebene Rezeptor eine Isoform des CFR-1 ist. Eine abweichende Zellverteilung von CFR-1 und seiner Homologe ist wahrscheinlich für die oben genannten Resultate verantwortlich und ist ein bekanntes Phänomen für andere Proteine (Smalheiser, 1996 Mol.Biol.Cell 7:1003-1014). Eine abweichende Verteilung kann durch ein abweichendes Glycosilierungsmuster in malignen Zellen verursacht werden, die zu einem Transport zu den Plasmamembranen führen.

Die Gewebeverteilung zeigt, daß das CFR-1 Molekül mit aktivierten und prolieferierenden Zellen in Verbindung steht, wie durch Anfärben mit dem Antikörper Ki67 gezeigt wurde (Ramires et al., 1997 J.Pathol 182:62-67). Die normale Magenschleimhaut zeigt diesen Rezeptor nicht in meßbarem Umfang, aber die mit H. pylori infiltrierte Epithelien oder displastische Epithelien weisen dieses Antigen auf. Beide Gewebe sind proliferativ und können Vorgänge für den Magenkrebs sein.

Für das Verständnis der hohen Wirksamkeit entscheidend ist, daß sich im Gegensatz zur Struktur des CFR-1, die sich auch auf gesunden Zellen findet, die ermittelte Isoform sich nicht auf gesunden sondern ausschließlich auf stark prolieferierenden Zellen findet, Zellen also, die sich stark teilen, wie beispielsweise die im Wachstum befindlichen Tumorzellen sowie entsprechender Vorläuferstufen. Die Wirkungsweise des Rezeptors beruht im wesentlichen darauf, daß er als Energierezeptor der Nahrungsaufnahme der Zellen dient und insbesondere bei Zellen mit häufiger Teilung, zu denen die Karzinomzellen zählen, einen dominierenden Anteil aufweisen. Ausdrücklich anzumerken ist, daß dieser Rezeptor nicht nur bei Magenkarzinomen Anwendung finden wird, sondern auch bei allen epithelialen Tumoren, die im wesentlichen gleiche Reaktionsmechanismen aufweisen. Hinausgehend über Magentumore wurde die Existenz dieser Rezeptoren in kanzerogenen Gewebe folgender Tumore nachgewiesen: Speiseröhre, Magen, Darm, Rektum, Leber, Gallenblase, Bauchspeicheldrüse, Lunge, Bronchien, Brust, Cervix, Prostata, Cardia, Barrett, Eierstock und/oder Uterus. Die tumorwirksamen Antikörper, die an den erfindungsgemäßen Rezeptor andocken, wirken also gezielt wirksam an den kanzerogenen (und nicht den gesunden) Zellen.

Die Glycoproteine der Rezeptorstruktur können über deren Molekulargewicht von etwa 130 kD identifiziert werden, wobei sich das Molekulargewicht in einer der bekannten Weise, so z. B. vermittels der Gelelektrophorese ermittelt werden kann. Der Begriff "etwa" bezieht sich darauf, daß für einen Fachmann erkennbar ist, daß derartige Größenbestimmungen keinesfalls exakt sind, sondern Veränderungen oder Variationen der Methoden der Molekulargrößenbestimmungen zu Schwankungen in der Meßwerten führen.

Das bedeutendste Anwendungsgebiet des Rezeptors ist das der Diagnose und Therapie. Bei der prophylaktischen Anwendung wird der Rezeptor dem Patienten in pharmazeutischen Dosen verabreicht, mit dem Ziel der Stimulierung von Antikörpern, so daß sich im Ergebnis eine Vaccinierung mit Hilfe des Rezeptors erreichen läßt. Die Antikörper tragen Sorge dafür, etwaig entstehende Tumorzellen zu beseitigen.

Aber auch die Verabreichung des Rezeptors bei bereits vorhandenen Tumorzellen ist eine Möglichkeit der Medikation. Die verabreichten Rezeptoren unterstützen und verstärken die Antikörperbildung und sorgen folglich für eine erhöhte Apoptose der Tumorzellen oder für eine komplemettvermittelte Lyse. Die Zelle "verhungert", da eine Blockierung des Rezeptors zu einem Wachstumsstillstand führt.

Die bisherigen Untersuchungen haben gezeigt, dass sich der Rezeptor insbesondere zur Bekämpfung der nachfolgend genannten Tumorvorstufen nachweislich eignet. Im Hinblick auf die Erkrankungen des Magens eignet sich der Rezeptor zur Bekämpfung der Dysplasie der Magenschleimhaut und/oder der intestinalen Metaplasie des Magens und/oder der Bekämpfung einer Magenschleimhautentzündung, die mit dem Bakterium Helicobacter pylori assoziiert ist sowie zur Bekämpfung tubulärer und tubulovillöser Adenome des Magens.

Eine Anwendung ist insbesondere auch bei folgenden Erkrankungen des Dickdarms angezeigt, nämlich der tubulären Adenome des Dickdarms, der villösen Adenome des Dickdarms und der Dysplasie bei Collitis ulcerosa.

Der Rezeptor ist gleichermaßen geeignet bei Barrett-Dysplasie und der Barrett-Metaplasie der Speiseröhre.
Auch zur Bekämpfung der nachfolgend genannten Erkrankungen des Gebärmutterhalses ist der Rezeptor geeignet:
Der cervicalen intraepithelialen Neoplasie I, der cervicalen intraepithelialen Neoplasie II und der cervicalen intraepithelialen Neoplasie III.

Schließlich eignet sich der vorbeschriebene Rezeptor auch zur Anwendung bei Plattenepithel Metaplasie und Platteneptihel Dysplasie des Bronchus.

Aufgrund der vorbeschriebenen Wirkmechanismen eignet sich der Rezeptor grundsätzlich zur Bekämpfung von Tumoren der Speisröhre, des Magens, Darms, der Rektums, der Leber, Gallenblase, Bauchspeicheldrüse, Lunge, Bronchien, Brust, Cervix, Prostata, Cardia, Barrett, Eierstock und/oder Uterus.

Die Verwendung des Rezeptors zu Diagnosezwecke nutzt die Bindefähigkeit der Antikörper an diesem Rezeptor aufgrund der spezifischen Antigen/Antikörper-Wechselwirkung. Auf diese Weise läßt sich über die Bindungsfähigkeit an den Rezeptor ein Nachweis für die Existenz, die Lokalisierung und/oder die Menge der entsprechenden Antikörper führen. Nach den gleichen Reaktionsmechanismen ist über die Bindefähigkeit ein Nachweis des Rezeptors möglich.

Insbesondere dann, wenn die Antikörper Tumorantikörper sind, können sie zum Nachweis für die Existenz von Tumoren genutzt werden. Insbesondere ist es möglich, den Rezeptor als Tumormarker zu nutzen.

In einer Weiterbildung kann der Rezeptor zur Herstellung eines Antitumormittels genutzt werden, in dem potentiell antitumorwirksame Substanzen auf ihre spezifische Bindefähigkeit an den Rezeptor untersucht werden und bei positiven Ergebnis, d.h. bei Eingehen einer Bindung, diese Substanz für die pharmazeutische Anwendung genutzt wird. Selbstverständlich ist eine entsprechende Konfektionierung und die Beigabe üblicher Zuschlagsstoffe zur Herstellung des in den Verkehr gelangenden Pharmazeutikums in der üblichen Weise erforderlich.

Ausdrücklich klarzustellen bleibt, daß für die Herstellung von Antitumorarzneien unter Zuhilfenahme des Rezeptors im vorbeschriebenen Sinne nicht nur humane Antikörper in Frage kommen, sondern auch Mausantikörper und/oder humanisierte Antikörper beliebiger Spezies. Gleichermaßen gilt dies für Antikörperfragmente wie z. B. Fab und F(ab)₂ und/oder Fab'-Fragmente, wie sie durch die proteolytische Spaltung von Antikörpern erhalten werden. Hierzu zählen weiterhin Einzelstrangantikörper und/oder tetramere und/oder dimere Antikörperform und/oder bispezifische Antikörper.

Weiterhin ist bekannt, dass humane Tumorantigene, die in Mäusen immunogen sind, zur Erzeugung monoklonaler Mausantikörpern verwendet werden, und die in der Lage sind, das humane Antigen spezifisch zu erkennen und deshalb geeignet sind, bei Menschen therapeutisch verwendet zu werden.

Aufgabe der zugrundeliegenden Erfindung ist die Ermittlung der Rezptorstruktur sowie deren Verwendung. Allerdings ergibt sich das Problem, dass die wiederholte Injektion von "fremden" Antikörpern bsp. Mausantikörpern in Menschen sowohl zu nachteiligen Hypersensibilitätsreaktion sowie erhöhten Clearence-Rate der zirkulierenden Antikörpern führen, sodass die Antikörper ihre Zielstelle nicht erreichen.

Aus diesen Gründen bedarf es eine Überprüfung der therapeutischen Verwendbarkeit von Mausantikörpern. Dessen ungeachtet ist die Verwendbarkeit im Zusammenhang mit diagnostischen Verfahren uneingeschränkt. Auch besteht die Möglichkeit, humanisierte Mausantikörper abzuleiten und zu therapeutischen Zwecken zu nutzen. Auch hier ist entscheidend, daß nicht nur die bereits vorliegenden Tumore sondern auch präkanzerogene Strukturen mit Hilfe dieser Diagnoseverfahren ermittelbar sind.

In der vorliegenden Erfindung wird Schutz beansprucht für einen an den vorbeschriebenen Rezeptor spezifisch bindenden Mausantikörper, dessen Struktur durch die Anlagen A, B definiert ist. Die für alle Antikörper identischen Regionen wurden nicht wiedergegeben; beansprucht und dargestellt wurden jene für den individuellen Antikörper charakteristischen Regionen.

Im Ergebnis erlaubt es der in seiner Struktur beschriebene Rezeptor, der als Isoform zu CFR-1 zu bezeichnen ist, eine Therapie und Diagnose nicht nur von Tumoren sondern auch präkanzerogene Strukturen. Darüber hinaus wird ein spezifischer hieran bindender Mausantikörper in seiner Struktur beschrieben.

### Materialien und Methoden

### Zellkultur und Antikörperreinigung

Für alle Untersuchungen wurde die bekannte Magenadenokarzinom-Zellinie 23132 (Hensel et al. 1999, Int.J.Cancer 81:229-235) verwendet. Die Zellen wurden bis zu 80% Konfluenz in RPMI-1640 (PAA, Wien Österreich) ergänzt mit 10% FCS und Penicillin/Streptomycin (1% für beide) gezüchtet. Für die beschriebenen Untersuchungen wurden die Zellen mit Trypsin/EDTA getrennt und zweifach mit Phosphat gepufferter Kochsalzlösung (PBS) vor Benutzung gewaschen.

Die menschliche Hybridomazelllinie 103/51 wurde gewonnen und gezüchtet wie beschrieben in Vollmers et al., 1994 Cancer 74:1525-1532. Die Reinigung der IgM Antikörper wurde in der Weise vorgenommen wie anderweitig bereits beschrieben (Vollmers et al., 1998 Oncol.Rep. 5:549-552).

### Präparation von Membranauszügen

Die Isolierung der Membranproteine aus Tumorzellen wurde in der Weise, wie sie durch Hensel et al. (Hensel et al., 1999, Int.J.Cancer 81:229-235) beschrieben wurde, unter Benutzung der Zelllinie 23132 duchgeführt. Verkürzt wiedergegeben wurden die zusammenhängenden Tumorzellen zweimal mit PBS gewaschen, mit einem Zellschaber abgelöst und zentrifugiert, und in einem hypotonischen Puffer (20 mM HEPES, 3 mM KCl, 3mM MgCl₂) aufgelöst. Nach einer 15 Min. Inkubationszeit auf Eis und einer Ultraschallbehandlung für 5 Min., wurden die Kerne durch Zentrifugieren bei 10.000 g für eine Dauer von 10 Min. pelletiert. Das Supernatant wurde für 30 Min. bei 100.000 g in einem Swing-out rotor zentrifugiert und hierdurch die Membran pelletiert. Nachdem die Pellets mit dem hypotonischen Puffer gewaschen wurde, wurden sie in einen Membran Lysis Puffer (50 mM HEPES pH 7.4, 0,1 mM EDTA, 10% Glycerol, and 1% Triton X-100) erneut aufgelöst. Ein Protease Inhibitor (Boehringer, Mannheim, Deutschland) wurde allen Lösungen zugeben.

### Western blotting:

Die Auftrennung durch 10 %ige SDS-PAGE Gele und Western blotting der Proteine wurden unter Standardbedienungen, wie anderweitig beschrieben (Hensel et at., 1999, Int.J.Cancer 81:229-235), durchgeführt. Verkürzt wiedergegeben wurden die geblotteten Nitrozellulose -Membrane mit PBS blockiert, welches 2 % Magermilchpulver enthielt, dem eine einstündigen Inkubation mit 10 µg/ml gereinigtem Antikörper 103/51 folgte. Nach dreimaligem Waschen mit PBS + 0,05% Tween-20 wurde der zweite Antikörper (Peroxidase gekoppelte Hasen antihumane IgM Antikörper (Dianova, Hamburg, Deutschland)) inkubiert. Die Reaktion wurde mit Hilfe des Supersignal Chemilumineszenz kits von Pierce (KMF, St. Augustin, Deutschland) nachgewiesen.

### Reinigung des Antigen 103/51

Die Reinigung des Antigens wurde mit Säulenchromatographie unter Verwendung einer Pharmazia (Freiburg, Deutschland) FPLC Einheit durchgeführt. Für die Größenausschluß-Chromatographie wurde eine Pharmazia Superdex 200 (XK 16/60) Säule mit 5 mg des Membranpräparates geladen und mit einem Puffer A betrieben (100 mM Tris/Cl, pH 7.5,2 mM EDTA, 40mM NaCl, 1% Triton X-100). Dann wurde das Eluat fraktioniert und durch Western blot Analyse auf Reaktionen mit Antikörpern 103/51 untersucht. Die positiven Fraktionen wurden auf einer MonoQ (5/5 Säule) unter Verwendung des Puffers A gegeben. Die gebundenen Proteine wurden mit Hilfe eines linearen Gradienten unter Verwendung des Puffers B (100 mM Tris/ Cl, pH 7.5,1 M NaCl, 2 mM EDTA, 1 M NaCl, 1 % Triton X-1 00) ausgewaschen, fraktioniert und mit Coomassiegefärbtem SDS-PAGE and Western blot Analyse untersucht. Die positiven Banden wurden aus dem Gel getrennt und sequenziert oder zur Immunisierung von Mäusen genutzt.

### MALDI peptide Aufzeichnung

Die interessierenden Banden wurden herausgetrennt und in kleine Stücke von etwa 1 mm x 1 mm zerschnitten. Die Gelstücke wurden gewaschen, mit DTT reduziert, S-Alkyliiert mit lodoacetamid und In-Gel aufgelöst mit Trypsin (unmodifiziert, Sequenzgrad, Boehringer) wie anderweitig beschrieben (Shevckenko et al., 1996b Anal.Chem. 68:850-858). Nach 3 Stunden der Verdauung bei 37°C wurden 0.3 µl der Lösung entfernt und einer MALIDI peptid Massenspektrometrie auf einem Bruker Reflex MALDI-TOF unterzogen, welches mit einer nachträglichen Extraktion (Brucker, Franzen, Bremen, Deutschland) ausgerüstet war. Die Dünnfilmtechnik wurde zur Probenpräparierung angewendet (Jensen et al., 1996 Rapid.Commun.Mass.Spectrom 10:1371-1378). Die tryptischen Peptid-Massen wurden dazu verwendet, um nicht redundante Proteinsequenzdaten durch ein Peptid-Suchprogramm, das im Hause entwickelt wurde, zu suchen.

### Klonen des CFR-1 Antisense-Vektor und Transfektion

RNA Isolierung, cDNA Synthese und PCR wurden wie beschrieben (Hensel et al., 1999 Int.J.Cancer 81:229-235) durchgeführt. Verkürzt wiedergegeben wurde für die Amplification von PCR aus einem 897 bp Fragment aus einem Bereich von 802 bis 1699 Basenpaare folgende Primer benutzt: CFR-für 5'
GCTTGGAGAAAGGCCTGGTGAA 3', CFR-Rev 5'
TGGCACTTGCGGTACAGGACAG 3'. Die Amplification wurde mit folgendem Zyklusprofil durchgeführt: 95°C, 2 Min; nachfolgend 35 Zyklen bei 94°C, 30 sec; 60°C, 30 sec; 72°C 60 sec. und abschließend 72 °C, 4 min. Die Klonierung in den pCR- Skript Amp SK (+) Vektor und das Sequenzieren der DNA wurde wie früher schon beschrieben (Hensel et al., 1999 Int.J.Cancer 81:229-235) durchgeführt. Der Insert wurde in den pHook-2 Vektor (Invitrogen, Leek, Niederlande) subkloniert und das Klonen wurde erneut durch Sequenzierung überprüft.
Die Transfektion der Zellinie 23132 mit pHook2-anti CFR-1 wurde mit einem Primefaktor Reagenz (PQLab, Erlangen, Germany) entsprechend dem Lieferantenhandbuch vervollständigt. In Kürze, dass Plasmid DNA wurde auf 10 µg/ml verdünnt und das Primefaktorreagenz im Verhältnis 1:10 einem serumfreien Wachstumsmedium beigegeben. Die verdünnte Plasmid DNA ( 450 µl), das verdünnte Primefaktorreagenz ergänzend (90 µl) und das serumfreie Wachstumsmedium (460 µl) wurden vermischt und bei Raumtemperatur inkubiert. 60 ml Zellkulturschalen (70% konfluent) wurden zweimal mit dem serumfreien Wachstumsmedium gewaschen und anschließend die Primefaktor/DNA-Mischung tropfenweise hinzugegeben. Die Zellen wurden inkubiert für 18 Stunden bei 37°C and 7% CO₂, anschließend wurde das serumfreie Wachstumsmedium ersetzt durch ein Wachstumsmedium mit 10% FCS und die Zellen wurden weitere 24 Stunden inkubiert, bevor die Expression der CFR-1 Struktur untersucht wurde.

### Durchflusszytometrie

Die Zelllinie 23132 wurde von den Kulturplatten durch Trypsin /EDTA 48 Stunden nach der Transfektion abgelöst, gewaschen und anschließend auf Eis mit dem Antikörper 103/51 oder dem humanen Isotyp-kontrol Antikörper (Chromopure human IgM) für 15 min, inkubiert, gefolgt von einer Inkubation mit einem FITC-gekoppelte Hasen Anti-Human IgM Antikörper (Dianova) für 15 Min. auf Eis. Die Antikörper wurden optimal in der 0,01 % Natriumazid PBS enthalten verdünnt. Die Zellen wurden mit Durchflusszytometrie (FACScan; Becton Dickinson, USA) analysiert.

### Glycosidaseassays

Abgelöste und gewaschene Zellen werden erneut in RPMI-1640, welches 10% FCS enthält, suspendiert und für 1 Stunde auf Eis inkupiert, anschließend gezählt und die Cytospins präpariert. Nach Lufttrocknung, Acetonfixierungen der Cytospins Präparate (10 Min), gewaschen, inkubiert mit 20 µU/ml O-Glycosidase oder 5 mU/ml N-Glycosidase (Boehringer) für 4 Stunden bei 37°C. Anschließend wurden die Objektträger gewaschen und immunhistochemisch gefärbt.
Für die Deglycosylation der Membran-Proteine wurden die Membranextrakte für 16 Stunden bei 37°C mit 1 mU/ml N-Glycosidase, verdünnt in Deglycosylation Puffer (50 mM POO-Buffer, pH 7,4) inkubiert. Zur Kontrolle wurden die Extrakte mit Deglycosylation Puffer allein inkubiert. Dann wurden die Extrakte durch SDS-PAGE separiert und die Western blots, wie oben beschrieben, durchgeführt.

### Herstellung murine monoklonaler Antikörper

BALB/cMäuse wurden zweimal innerhalb 17 Tage mit 5 µg gereinigten Antigen des Antikörpers 103/51 immunisiert, und 4 Tage nach der zweiten Immunisierung getötet. Die Milz wurde mechanisch entfernt und mit 1 x 10⁷ NSO Zellen, wie frührer schon beschrieben (Vollmers et al,. 1985 Cell 40:547-557), verschmolzen. Die Antikörper produzierenden Hybridome wurden durch immunohistochemische Färbung und der Reaktion in der Western blot Analyse getestet. Der Klon 58/47-69 mit positiver Reaktion wurde für die weiteren Experimente genutzt.

### Immunohistochemisches Färben der Paraffin Sektionen

In Paraffin eingebettete menschliche Magenschleimhaut mit Tumorzellen wurde zerkleinert (5µg), entparaffiniert, und blockiert mit BSA (15 mg/ml), welches in PBS verdünnt ist, für 30 Min. Die Sektionen wurden inkubiert mit dem Überstand der Hybridoma Zellen 103/51 oder 58/47-69, Ki 67 (Loxo, Dossenheim, Deutschland) oder Mausanti-cytokeration 8 Antikörper, verdünnt auf 1:15 mit BSA/PBS (Dako, Hamburg, Deutschland) für 2 Stunden in einem feuchten Inkubator. Anschließend wurden sie dreimal Tris/NaCl, gewaschen, gefolgt durch eine Inkubation mit Peroxidase-gekoppelten Hasen antihuman oder Hasen Antimaus-Konjugate (Dako), verdünnt auf 1:50 in PBS enthaltend ein Hasenserum (für den Antikörper 103/51) oder in PBS enthaltend menschliches AB Plasma (für Antikörper 58/47-69 und Anti-Cytokeratin). Nach dreimaligen Waschen mit Tris/NaCl und Inkubation in PBS für 10 Min. wurde das Färben mit Diaminobenzidine (0,05%)-Hydrogen Peroxyd (0.02%) für 10 Min. bei Raumtemperatur durchgeführt. Die Reaktion wurde unter laufendem Wasser gestoppt und die Sektionen wurden mit Hematoxylin gegengefärbt.

### Immunohistochemisches Färben von lebenden und Aceton fixierten Zellen

Für das Färben lebender Zellen wurden die Zellen herausgelöst, gewaschen und verdünnt auf 1 x 10⁶ Zellen pro ml. 1 ml der Zelllösung werden bei 1.500 g für 5 Min. zentrifugiert. Der auf 40 µg/ml mit vollständigen RPMI verdünnte Antikörper wird zu einem Endvolumen von 1ml ergänzt und für 90 Min. auf Eis inkubiert. Dann werden die Zellen bei 1.500 g für 5 Min. pelletiert und wieder aufgelöst mit 500 µl RPMI. Mit 200 µl der Zelllösung werden die Zytospinpräparate präpariert und für 30 Min luftgetrocknet. Die Zellen werden in Aceton für 30 Min. fixiert und dreimal mit Tris/NaCl gewaschen. Die HRPgekoppelten Hasen antihumanen IgM (DAKO) werden 1:50 in PBS/BSA (01,%) verdünnt und für 30 Min. bei Raumtemperatur inkubiert. Nach dreimaligem Waschen wird das Färben, wie oben erwähnt, durchgeführt.
Für das Färben der azeton-fixierten Zellen werden die Zytospins präpariert, bei Raumtemperatur luftgetrocknet und, wie oben beschrieben, in Azeton fixiert. Dann werden die Zytospins für 15 Min. mit PBS/BSA (0,1 %) blockiert und für 30 Min. mit 10 µg/ml primärer Antikörper inkubiert und anschließend dreimal gewaschen. Die Inkubation mit den sekundären Antikörpern und das Färben wird, wie oben beschrieben, durchgeführt.

### MTT-Proliferation Untersuchung

Die MTT- Untersuchung der bekannten Zelllinie 23132 wurde wie beschrieben (Vollmers et al., 1994 Cancer 74:1525-1532) durchgeführt. Verkürzt wiedergeben wurden die trypsinizierten Zellen mit Wachstumsmedium auf 1 x 10⁶ Zellen/ml verdünnt und eine 50 µl Zellsuspension wurde jeder Vertiefung eine 96-Loch Zellkulturplatte zugegeben. Dann wurden 50 µl der Antikörper, verdünnt in den angegebenen Konzentrationen mit Wachstumsmedium, zu diesen Vertiefungen addiert und die Platten wurden für ein oder zwei Tage bei 37° C in einen feuchten Inkubator inkubiert. Zur Messung wurden 50 µl auf MTT (3(4,5 Dimethylthiazol)-2,5 Diphenyltetrazolium bromid) Lösung (5 mg/ml) in jede Vertiefung gegeben und die Platten wurden für 30 Min. inkubiert. Nach der Inkubation wurden die Platten bei 800 g für 5 Min. zentrifugiert, die MTT Lösung entfernt und das gefärbte Zellpellet in 150 µl Dimethylsulphoxid auflöst und die Absorption bei einer Wellenlänge von 540 nm und 690 nm gemessen.

### Methoden zur Bestimmung der Sequenz von CFR-1

Die Präparation von RNA für die cDNA-Synthese erfolgte mit Hilfe des RNeasy-Kits von Quiagen. Zur Vorbereitung wurden 1x10⁶ Zellen zweimal mit eiskaltem PBS gewaschen und mit 1.000 x g für 5 min pelletiert und entsprechend der Herstellerbeschreibung die RNA präpariert. 5 µg RNA (1-5 µl Lösung) wurden mit 1 µl Oligo-dT₁₅ (1 µg/µl) und 2 µl random primer (40 µM) gemischt und auf ein Gesamtvolumen von 8 µl mit H₂O aufgefüllt. Es erfolgte die Denaturierung der RNA für 10 min bei 65°C und anschließend die Abkühlung der Probe auf Eis. Zu dieser wurden dann 17 µl Mastermix, bestehend aus 5,2 µl DEPC-H₂O, 5 µl 5fach Reverse-Transkriptase-Puffer, 2,5 µl dNTPs (je 10 mM), 2,5 µl DTT (250 mM), 0,8 µl RNasin (400 U) und 1 µl M-MLV Reverse Transkriptase (200 U) pipettiert. Die Synthese der cDNA erfolgte für 70 min bei 37°C und wurde anschließend durch eine Erhitzung auf 95°C für 5 min abgebrochen. 1-5 µl der cDNA wurden mit dem PCR-Mastermix gemischt und auf 25 µl Gesamtvolumen mit H₂O aufgefüllt. Der PCR-Mastermix besteht aus 2,5 µl 10fach Taq-Polymerase-Puffer, 0,5 µl 10 mM NTPs, 1,5 - 2 µl 25 mM MgCl₂, je 0,5 µl 20 pM 3' bzw. 5' Primer und 0,2 µl Taq-Polymerase (1 U). Die Amplifikationsbedingungen für die verschiedenen PCR-Produkte sind in der folgenden Tabelle aufgeführt.

Übersicht der zur Ampflikation der verschiedenen cDNAs verwendeten PCR-Programme

| Produkt | Annealing in [°C] | MgCl₂ [mM] | Extensionszeit [sec] | Zyklen | Produktgröße [bp] |
|---|---|---|---|---|---|
| Fragment 1 | 55 | 1,75 | 45 | 40 | 691 |
| Fragment 2 | 60 | 1,5 | 45 | 40 | 898 |
| CFR Fragment 3 | 55 | 2,0 | 45 | 40 | 739 |
| Fragment 4 | 55 | 2,0 | 45 | 40 | 941 |
| Fragment 5 | 55 | 2,0 | 45 | 40 | 750 |

### Primer-Sequenzen

Sequenzen der für die PCR verwendeten Oligonukleotide

| CFR | | | |
|---|---|---|---|
| CFR-For 1 | 5' | OGC AGC TTC AGC AGC AAC AGC A | 3' |
| CFR-Rev 1 | 5' | CAG CTC AGC CAC CCG GAG AAT G | 3' |
| CFR-For 2 | 5' | GCT TGG AGA AAG GCC TGG TGA A | 3' |
| CFR-Rev 2 | 5' | TGG CAC TTG CGG TAC AGG ACA G | 3' |
| CFR-For 3 | 5' | GAA CAC CGT CTC TTA GAG CTG C | 3' |
| CFR-Rev 3 | 5' | GCT TCC TGC AGA GTG TCA TTG C | 3' |
| CFR-For 4 | 5' | GGA GGA CGT GTT GAA GCT TTG C | 3' |
| CFR-Rev 4 | 5' | CCA GGG CAC AAG CAG TAT GAA G | 3' |
| CFR-For 5 | 5' | CAA CAG CAG ACA GGT CAG GTG G | 3' |
| CFR-Rev 5 | 5' | CCG GAA GTT CTG TTG GTA TGA G | 3' |

Die Sequenzierung erfolgte mit einem Sequenzierautomaten der Firma AppliedBiosystems. Für die Sequenzierung klonierter PCR-Produkte wurden die folgenden Oligos verwendet:

| | | | |
|---|---|---|---|
| T₃ | 5' | ATT TAA CCC TCA CTA AAG GG | 3' |
| T₇ | 5' | GTA ATA CGA CTC ACT ATA GGG C | 3' |

3 µl Plasmid-DNA, isoliert wie unter 2.4.15. beschrieben, wurden mit 1 µl Primer (3,2 pM), 11 µl H₂O und 5 µl Reaktionsgemisch des Abi-Prism Sequencing Kits gemischt und im Thermocycler für 25 Zyklen mit den folgenden Parametern inkubiert:

| Denaturierung | Annealing | Extension |
|---|---|---|
| 95°C, 30 sec | 52°C, 15 sec | 60°C, 4 min |

Zum Entfernen von Oligos und dNTPs wurde das Reaktionsgemisch über eine Sephadex G-50 Säule gereinigt. Hierzu wurde eine 100 µl Pipettenspitze bis zum oberen Rand mit Säulenmaterial beladen und für 3 min bei 2.000 x g zentrifugiert. Anschließend wurde die Probe aufgegeben und das Säulchen nochmals zentrifugiert. Die DNA wurde dann durch 2 µl Na-Azetat (pH 5,2) und 50 µl 100% Äthanol ausgefällt und durch eine Zentrifugation bei 13.000 x g für 15 min pelletiert. Nach dem Trocknen wurde die DNA in 3 µl Formamid/25 mM EDTA (5:1) aufgenommen und im Sequenzierautomaten analysiert.

### Auswertung der Sequenzierungen

Von allen Klonierungen wurden mindestens fünf Klone sequenziert. Um Fehler zu beseitigen, die bei der Amplifikation mit der Taq-Polymerase bzw. der Sequenzierung entstanden sind, wurden die Sequenzen der klonierten PCR-Fragmente mit Hilfe der DNAsis für Windows Software untereinander verglichen und eine Konsensus-Sequenz aller Klone aus beiden Leserichtungen erstellt. Durch Umschreiben der DNA-Sequenzen in Aminosäure-Sequenzen wurde dann die Anzahl der stillen Mutationen und der Aminosäureaustauschmutationen bestimmt. Die Sequenzen für MG160 und CFR wurden aus der NCBI-Datenbank bezogen und mit dem DNAsis für Windows Programm mit Sequenzierungen der PCR-Produkte verglichen.

### Figuren und Tabellen

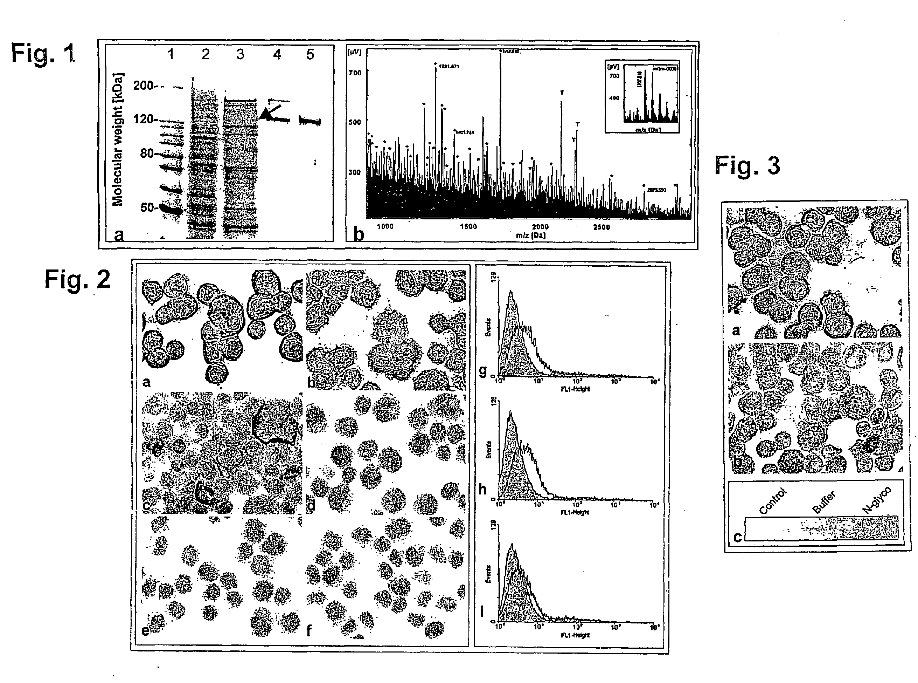

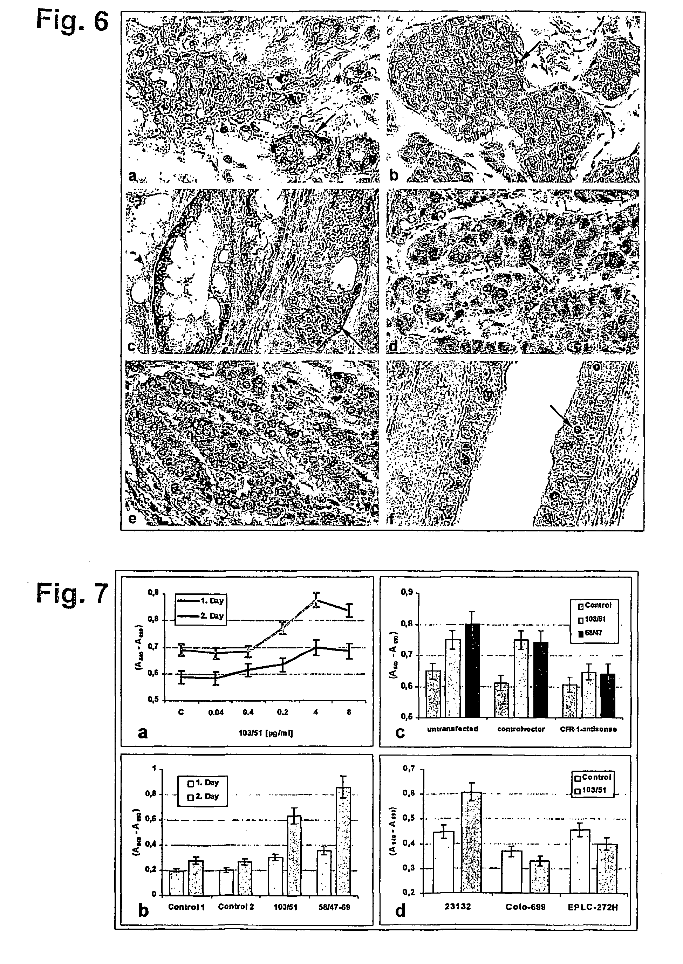

**Figur 1****:** Identifizierung des Antigens des Antikörpers 103/51
   a) Proteinreinigung des Antigens aus Membranextrakten der Magenkrebszellinie 23132. Die Membranfraktionen werden chromatographisches Verfahren unterworfen und Ganzmembranfraktionen (Reihe 2) oder gereinigte Proteine (Reihe 3) werden mit Coomassie eingefärbt (Reihe 1 : 10 kDa Tabelle). Die Western Blot Analyse mit dem Antikörper 103/51 auf Membranfraktionen der Zellinie 23132 angewendet zeigt eine Reaktion mit einem Protein mit Molekulargewicht von näherungsweise 130 kD (Reihe 4). Spezifitäten der gereinigten Membranextrakte wurde durch Westernblotting mit 103/51 kontrolliert (Reihe 5). Das durch einen Pfeil gekennzeichnete Protein wurde aus präparativem Gel gewonnen und für MALDI Massenspektromtrie und die Immunisierung von Mäusen genutzt.
   b) Identifizierung der 130 kDa gelseparierten Proteine durch hochauflösende MALDI peptid Massenspektrometrie. Die mit "*" bezeichneten Spitzen geben die berechneten Massen der tryptischen Peptide des U28811 humanen systeinreichen fibroblast growth factor receptor (CFR-1) mit einer Massengenauigkeit von mehr als 50 ppm wider. Die mit "T" bezeichneten Spitzen entsprechen den Autolyseprodukten des Trypsin. Der Einschub zeigt eine Massenauflösung (m/Δm = 9000) der Spitzen bei m/z 1707.818.
**Figur 2****:** Einfluß der CFR-1 Antisense-Transfektion auf Färbungen von Antikörpern 103/51 und Lebendzellfärbung (Vergrößerung 200x).
   a) Die Zellinie 23132 wurde mit Kontrollvektor vorübergehend transfiziert und die Azetonfixierung zeigt eine intensive Färbung mit Antikörper 103/51.
   b) Eine reduzierte Einfärbung von kurz mit CFR-1 Antisensevektor transfektierten Zellen ist sichtbar.
   c) Um den Hintergrund im immunohistochemischen Färbungen zu verringern, wurden Lebendzelle der Zellinie 23132 eingefärbt. Eine deutliche Membranfärbung ist sichtbar.
   d) Zur Kontrolle eine Lebendzellfärbung (nur sekundäre Antikörper) der Zellinie 23132.
   e) Das negative Lebendzellfärbung der Zellinie Colo-699 mit dem Antikörper 103/51 zeigt, daß diese Zellinie für CFR-1 negativ ist.
   f) Zur Kontrolle Lebendzellfärbung (nur sekundäre Antikörper) mit der Zellinie Colo-699.
   g) Durchflußzytometrie mit der Zellinie 23132 mit Antikörpern Chromopur human IgM (grau) und 103/51.
   h) Analyse von Zellen, die mit dem Kontrollvektor pHOOK-2 transfiziert sind, mit Durchflußzytometrie 48 Stunden nach der Transfektion.
   i) Zellen, die mit dem CFR-1 Antisense-Vektor transfiziert sind, zeigen einen deutlichen Abfall in der Bindung des Antikörpers 103/51.
**Figur 3****:** Einfluß der Deglycosylation auf die Färbung mit Antikörper 103/51
   a) Zellen (23132) mit Deglycosylationspuffer inkubiert und Azeton fixiert zeigen eine intensive Färbung mit Antikörper 103/51.
   b) Zellen (23132), die mit N-Glycosidase behandelt und anschließend Azeton fixiert wurden, zeigen eine deutliche Verringerung der Färbung.
   c) Der Einfluß des Declycosylation auf Membranextrakte der Zellinie 23132 in Reaktion mit dem Antikörper 103/51 in Westernblot-Analyse. Die für 16 Stunden mit Declycosylationspuffer (Puffer) inkubierten Extrakte zeigen keinen Unterschied im Färben im Vergleich zu unbehandelten Extrakten (Kontrolle). Die Inkubation mit N-Glycosydase führt zu einer deutlichen Verminderung im Färbung (N-Glyco).
**Figur 4****:** Immunohistochemische Färbung mit murinen Antikörpern 58/47-69 und 103/51 auf Magenadenokarzinomen Um identische Spezifizität des Antikörpers 103/51 und des murinen Antikörpers 58/47-69 zu zeigen, wurde Magenadenokarzinomzellen vom diffusen Typ eingefärbt mit Hämatoxilin-Eosin (a), Antikörpern 103/51 (b) und 58/47-69 (c), und Antizytokeratin 18 als positive Kontrolle. Die identische Färbung in c und d zeigt identische Spezifität (Pfeile = Tumorzellen).
**Figur 5****:** Immunhistochemische Färbung des Antikörper 103/51 auf verschiedenen Magengeweben
   Cryosektionen von Magengeweben werden mit HE, Antikörper Ki67 (um die proliferierenden Zellen anzuzeigen) und Antikörper 103/51 gefärbt (Vergrößerung x100)
   a) Magengewebe mit Entzündungen
   b) H. pylori induzierte Gastritis (die Einschübe zeigen eine Vergrößerung der markierten Drüsen)
   c) Dysplasi
   d) Magenadenokarzinom
**Figur 6****:** Immunhistochemisches Färbung mit Antikörper 103/51 auf verschiedenen kanzerogenen und normalen Geweben Das Färben der Antikörper 103/51 wird bei folgenden Geweben gezeigt: Karzinom des Vater-Gefäßes (a), lobulär invasives Brustkarzinom (b), Adenokarzinom des Darmes und keine Färbung der normalen Becherzelle der Darmschleimhaut (c), Leberzellenkarzinom (d), glomerulare und fasciculare Bereiche der Nebennierenrinde (e), Sammelgefäße der nierenspezifischen Färbung des Golgi-Apparates (Pfeil) (f). Die Pfeile in a - d zeigen Tumorzellen, der rote Pfeil in (c) = Becherzellen, der Pfeil in (f) deuten den Golgi-Apparat an (Vergrößerung 400x, mit Ausnahme (g) 200x).
**Figur 7****:** Stimulation der Zellinien mit Antikörpern 103/51 und 58/47-69 durch kolorimetrische MTT-Untersuchung bestimmt
   a) Die Titration mit gereinigtem Antikörper 103/51 zeigt ein Anwachsen der Stimulation bis zu 4 µg/ml. Höhere Konzentrationen führen nicht zu einer höheren Stimulation (c = Control, keine Zugabe von Antikörper).
   b) Eine MTT-Untersuchung mit gleichen Konzentration (4 µg/ml) von gereinigten Antikörpern 103/51 und 58/47-69 zeigen vergleichbare Stimulationen der Tumorzellinien 23132 nach ein oder zwei Tagen Inkubation (Kontrolle 1 = Chrompur humanes IgM, Kontrolle 2 = unkorreliertes Maus IgM).
   c) Die Zellinie 23132 wurde vorübergehend mit dem Kontrolvektor pHOOK-2 oder CFR-1 Antisense-Vektor transfiziert, für 24 Stunden inkubiert und in einer MTT-Untersuchung nach Stimulation mit 4 µg/ml gereinigtem Antikörper 103/51 nach 24 Stunden getestet. Die nicht transfizierten Zellen wurden ebenso zur Kontrolle inkubiert (Kontrolle = nichtkorrelierter humaner IgM).
   d) Eine MTT-Untersuchung mit gleichen Konzentrationen (4 µg/ml) des Antikörpers 103/51 auf unterschiedlichen Epithelialtumorzellinien zeigen eine Stimulation nur der CFR-1 positiven Zellinien 23132 24 Stunden nach Beigabe des Antikörpers. Die CFR-1 negativen Zellinien Colo-699 und EPLC-272H zeigen keine Stimulation durch den Antikörper 103/51.

### Tabelle 1: Reaktionsmuster des Antikörpers 103/51 mit verschiedenen Geweben

Die Färbung mit Antikörpern wurde wie folgt bewertet:
- = kein Färben, + = mäßiges Färben, ++ = intensives Färben. HCC = Hepatozellenkarzinom (Leberzellenkrebs), ¹Proliferationsbereich, Drüsenvertiefung, ²Glomerular, Fascicularbereich (Membranfärben), ³Sammelgefäße des retikulären Endothelgewebes.

### Anlage A

### Anlage B

Anlage S: Vergleich der Aminosäuresequenz der aus der Zellinie 23132 gewonnenen CFR-1 mit den bereits veröffentlichten Sequenzen von CFR-1 und MG160.

Diese Vergleichsversuche beweisen zunächst, daß das aus der Zellinie 23132 gewonnene CFR-1-Protein nicht identisch ist mit den vorbekannten CFR-1-Sequenzen sondern demgegenüber eine Isoform darstellt. Neben den Unterschieden gegenüber den vorbekannten und veröffentlichten CFR-1 und MG160 ist die Aminosäuresequenz einer speziellen Ausführungsform des allgemein beanspruchten Rezeptors abgesehen und von dem ersten und speziell gekennzeichneten Positionen eindeutig festgelegt.

### RESULTATE

### Reinigung und Identifizierung des Antigens 103/51

Die Western Blot Analyse wurde benutzt, um zu zeigen, daß der Antikörper 103/51 an ein etwa 130 kD Membranprotein auf einer Magenkrebszelle bindet. Die Vorreinigung dieses Proteins erfolgte durch sequenzielle Größenausschluß- und Anionenaustauschchromatographie (Fig. 1a). Das Protein wurde gewonnen aus einer Coomassie-gefärbten präparativen SDS-PAGE, ein Anteil hiervon wurde benutzt, um monoklonale Mausantikörper herzustellen (siehe unten) und der andere Teil wurde benutzt, um das Protein nach dem Verfahren, wie es Shevchenko et al. (1996 Proc.Natl.Acad.Sci.U.S.A. 93:14440:14445) beschrieben hat, zu identifizieren. Nach 3 Stunden in Geldigestieren mit Trypsin, wurden ca. 1% des insgesamt digestierten Volumens entfernt und einer hochgenauen MALDI peptid Massenspektrometrie (der Rest wurde für eine Nanoelektrosprayanalyse aufbewahrt, da eine MALDI MS nicht zu einer definierten Identifikation führte). Trotz des im Femtomolbereich liegenden Verbrauches des Proteindigestes für die MALDI-Analyse ergab die Untersuchung 35 Peptide der CFR-1 Sequenz mit einer Massengenauigkeit innerhalb von 50 ppm. Diese Peptide bilden 29% der CFR-1 Sequenz und identifizieren das Protein auf diese Weise das Protein, das ein errechnetes Molekulargewicht von etwa 134 kD hat (Burrus et al., 1992 Mol.Cell Biol. 12:5600-5609) (Fig. 1 b).

### Der Effekt der vorübergehenden Transfektion der Zellinie 23132 mit dem CFR-Antisense-Vector auf die Färbung des Antikörpers 103/51 und Färbung von Lebendzellen

Wir untersuchten den Effekt der Antisensetransfektion der Magenkrebszellinie 23132 unter Benutzung der Immunohistochemie und Durchflußzytometrie. Zu diesem Zweck wurde ein 897 bp PCR-Fragment des CFR, aus dem Bereich der Basenpar 802 und 1699 entnommen, mit dem pHOOK-2-Vector im Antisensesinne im Bezug zum CMV promoter geklont. Die gewaschenen Zellen wurden in einen Zwischenschritt mit dem pHOOK-CFR Antisensevector pHOOK-lacZ und pHOOK Vektor transfiziert. Die Transfektion wurde durch eine β-Galatosidase Untersuchung (Daten nicht gezeigt) überprüft. 48 Stunden nach der Transfektion wurden Cytospins präpariert und mit Antikörpern 103/51 und Anti-Zytokeratin 18 zur Kontrolle gefärbt (Daten nicht gezeigt).

Die Immunohistochemie zeigt eine deutliche Reduzierung der Zellfärbung, die mit pHOOK-CFR Antisense Vektor transfektiert sind, im Vergleich mit Kontrollzellen (Fig. 2 a - b). Das bestätigt die Bindung des Antikörpers 103/51 an CFR-1. Das leichte zytoplasmatische Färben, das in beiden Färbeprozessen zu sehen war, kann seine Ursache in nichtspezifischen Bindungen haben, wie sie oft beim Färben von menschlichen IgM Antikörpern auf azetonfixierten Zellen beobachtet wird. Die Zellexpression als auch der Einfluß der Transfektion wurden durch Durchflußzytometrie überprüft (Fig. 2 g - i). Die Daten zeigen eine Reduzierung von Bindungen des Antikörpers 103/51 nach Transfektion von Zellen mit dem CFR-1 Antisense Vektor. Jedoch zeigen unbehandelte Zellen oder Zellen, die mit den Kontrollvektor pHOOK-2 transfektiert wurden, eine deutliche Bindung zur Zellinie 23132, was die Expression von CFR-1 auf der Zellenmembran zeigt.

Um die spezifische Membranverteilung der CFR-1 Isoform zu untersuchen, färbten wir lebende Zellen der Zellinie 23132 und einiger Nichtmagenkrebszellinien. Auf der Zellinie 23132 ergab sich ein deutliches Färben (Fig. 2 c, d), während menschliche Lungenkarzinomzellinien Colo-699 (Fig. 2 e, f) und EPLC-272H (Daten nicht gezeigt) eindeutig negativ waren. Diese Daten zeigen, daß die beschrieben CFR-1 Isoform nicht in allen Krebszellinien vorhanden ist und ausschließlich das Einfärben der Membrane von 23132 Zellen zeigt, daß die CFR-1 Isoform eine Verteilung hat, die von der bislang für CFR-1 beschriebenen verschieden ist.

### Glycosidaseassay

CFR-1 ist ein Sialoglycoprotein mit 5 möglichen N-Glycosylation-Armen und es wurde durch Behandlung mit Glycosidase F gezeigt, daß das Molekül an diesen Stellen glycosoliert (Steegmaier et al. Nature 373:615-620, 1995). Da tumorreaktive Antikörper oft mit Carbohydrat-Gruppen reagieren, haben wir untersucht, ob dies auch für den Antikörper 103/51 der Fall ist. Cytospinpräparationen der Zellinie 23132 wurden für 4 Stunden mit O- und N-Glycosidase inkubiert und anschließend einem immunhistochemischen Färben mit dem Antikörper 103/51 unterworfen. Die Behandlung der Zelle mit N-Glycosidase führte zu einer dramatischen Verringerung in der 103/51 Färbung (Fig. 3 b), während die Inkubierung mit Dephosphorylation Puffer (Fig. 3 a) oder die Bearbeitung mit O-Glycosidase (Daten nicht gezeigt) keine Auswirkung auf die Bindung des Antikörpers 103/51 haben. Dies zeigt, daß die Bindungsspezifizität des Antikörpers 103/51 in den Zuckerbestandteilen und nicht in den primären Proteinsequenzen lokalisiert sind. Zur weiteren Überprüfung dieses Effektes wurden die Membranextrakte der Zellinie 23132 für 16 Stunden deglycosyliert und Western Blots durchgeführt und mit Antikörpern 103/51 gefärbt. Wir fanden eine Verringerung der Reaktion mit Lysaten, die mit N-Glycosidase inkubiert waren, im Vergleich zu den Kontrollysaten (Fig. 3 c).

### Herstellung muriner Antikörper und immunhistochemische Färbung von Paraffinschnitten des Magenkarzinoms

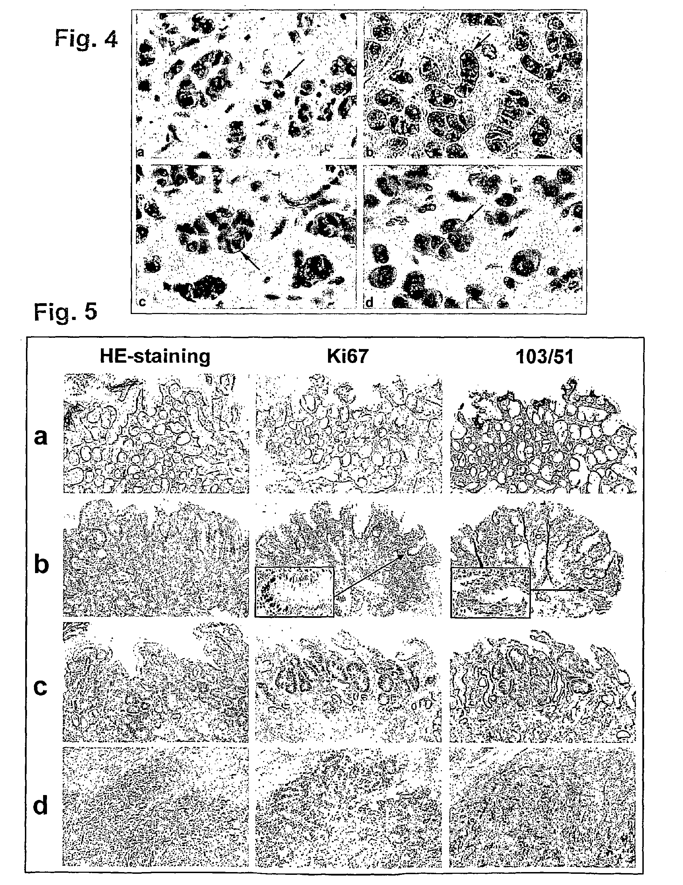

Da Antikörper gegen CFR-1 nicht käuflich erwerbbar sind, wurden Mäuse mit gereinigtem Protein, welches aus Coomassie gefärbten SDS-Gel ausgewaschen wurde, für die Produktion monoklonaler Antikörper immunisiert um die Spezifität zu bestätigen und weiterhin die CFR-1 Expression zu charakterisieren. Milzzellen wurden durch Fusion mit Heteromyelomzellen NSO konserviert. 150 Klone wurden durch immunhistochemisches Färbungen getestet. Die positiven Klone wurden erneut geklont und das Klon 58/47-49 (IgM) wurde zur weiteren Charakterisierung genutzt. Um die Bindungseigenschaften des humanen Antikörpers 103/51 und des murinen Antikörpers 58/47-69 zu untersuchen, färbten wir die Paraffinschnitte von 15 verschiedenen Magenadenokarzinom- und einem Adenozellen. Identisches Färben der Drüsenzellen der normalen Epithelialgewebe und intensives Färben der Karzinomzellen wurde erhalten (Fig. 4). Verkürzt wiedergegeben wurden die ersten Karzinomgewebe (n = 2) mit beiden Antikörpern gefärbt. Bei Darmkarzinomzellen haben beide Antikörper 4 von 5 Fällen angefärbt, bei diffusen Karzinomgeweben wurden alle Fälle (n = 4) gefärbt und bei Zwischentypen waren 50% (n = 4) bei beiden Antikörpern positiv. Diese Ergebnisse zeigen eine hohe Expressionsrate von CFR-1 in den meisten Fällen des Magenkarzinomgewebes. Das untersuchte Adenomagewebe zeigte ein bestimmtes Färbemuster mit positiven Zellen, nur im Übergang von den normalen zu den transformierten Zellen.

### Immunohistochemisches Färben mit Antikörper 103/51 auf Magenschleimhaut

Um die Reaktionen des Antikörpers 103/51 auf Magenschleimhaut näher zu untersuchen, haben wir ein immunohistochemische Färbungen von Magengewebe ohne Entzündung, durch H. pylori bedingte chronisch aktive Gastritis, hochgradige Dysplasie und Magenadenokarzinom durchgeführt. Bei dem nichtentzündeten Magengewebe wurden keine Reaktionen beobachtet (Fig. 5). Jedoch fanden wir in der Magenschleimhaut eines Patienten mit H. pylori Gastritis eine Einfärbung primär im Basalbereich der Foveolarzellen. Das Färbemuster des Antikörpers 103/51 zeigt einen strengen Zusammenhang mit dem Aktivierungsmuster, wie es sich beim Ki67-Färben zeigt (Ramires et al., 1997 J. Pathol 182:62-67). Eine intensivere Färbung des Antikörpers 103/51 konnte im Proliferierungsbereich der Magendysplasi gesehen werden, das ebenso Übereinstimmung mit dem Ki67-Färbung zeigt. Die stärkste Färbung wurde in den Proliferationsbereich der Magenadenokarzinoma gefunden.

### Immunohistochemisches Färben der Antikörper 103/51 und 58/47-69 auf verschiedenen Geweben

Wir untersuchten die Expression von CFR-1 in anderen kanzerogenen und normalen Geweben durch immunhistochemische Färbung auf Paraffinschnitten mit den Antikörpern 103/51 und 58/47-69. Bei 15 Krebsgeweben (verschiedener Magenkrebsarten) zeigte der Antikörper 103/51 ein Färben in 13 Fällen (Fig. 6, Tab. 1a). Ein negatives Ergebnis wurde bei den anaplastischen Zellen der Lunge beobachtet, was die Ergebnisse des immunhistochemische Färbung und der MTT-Untersuchung mit den Zellinien Colo-699 und EPLC-272H bestätigt. Diese Daten zeigen eine starke Expression auf CFR-1 und eine Verteilung in den Zellmembranen der durch Geschwulste veränderten Zellen.

Bei den 28 untersuchten normalen Geweben wurde eine geringe Expression nur in 3 Darmproben gefunden (Tab. 1 b). Eine Färbung der Membrane wurde bei Foveoladrüsen des Magens und den glomerularen und fascicularen Bereiche der Drüsenzellen beobachtet, wohingegen ein Färben des Golgi-Apparates in den Sammelröhren der Niere gefunden wurde (Fig. 5). Dies bestätigt weiterhin die Charakterisierung des Antigens als CFR-1, wie es schon früher durch Burrus et al. (1992) Mol.Cell Biol. 12:5600-5609 beschrieben wurde.

### Stimulierung mit humanen und murinen monoklonalen Antikörpern

Wie bereits in früheren Publikationen (Vollmers et al., 1994 Cancer 74:1525-1532; Hensel et al., 1999 Int.J.Cancer 81:229-235) erläutert, führt der Antikörper 103/51 zu einer Stimulation der Zellinie 23132 in vitro. Wir haben diese Stimulation des Antikörpers 103/51 durch Anwendung der Mitochondrialhydroxylaseuntersuchung (MTT) gemessen, welches eine Standarduntersuchung für Proliferation darstellt (Carmichael et al., 1987 Cancer Res. 47:936-942). Um weiterhin die Stimulationseigenschaften des Antikörpers 103/51 zu untersuchen, haben wir die Zellinie 23132 mit verschiedenen Konzentrationen gereinigter Antikörper inkubiert. Wir fanden eine konzentrationsabhängige Stimulierung mit höchster Aktivität bei 4 µg/ml (Fig. 7 a). Höhere Konzentrationen zeigten ein leichtes Abfallen in der Stimulation.

Um zu untersuchen, ob der murine Antikörper 58/47-69 den gleichen Einfluß auf das Zellwachstum hat, haben wir die MTT-Stimulationsuntersuchung mit gereinigten Antikörpern in vergleichbarer Menge durchgeführt. Wie aus Fig. 7 b entnommen werden kann, führten beide Antikörper zu einer Stimulation der Zellinie 23132 in vitro. Dies bestätigt weiterhin die identische Spezifität beider Antikörper.

Um zu bestätigen, daß die Stimulation des Antikörpers 103/51 und des murinen Antikörpers 58/47-69 durch die Bindung an CFR-1 vermittelt wird, haben wir die Zellen mit dem Kontrollvektor pHOOK-2 und dem CFR-1 Antisense-Vektor transfiziert und die Zellen durch eine MTT-Untersuchung getestet. Als positive Kontrolle für die Transfektion wurden die Zellen ebenso mit den pHOOK-2-lacZ Vektor transfiziert und danach mit β-Galactosidose eingefärbt (Daten nicht gezeigt). Da vergleichbare Stimulationen in den nichttransfektierten Fällen und den Zellen, die mit dem Kontrollvektor pHOOK-2 transfektiert wurden, beobachtet werden konnte, kann eine Reduzierung des Stimulationseffektes durch den Transfektionsprozeß ausgeschlossen werden. Im Gegensatz hierzu zeigten die Zellen, die mit dem CFR-1 Antisense-Vektor transfiziert wurden, eine verringernde Stimulation (Fig. 7 c).

Schließlich wurde, um zu zeigen, daß die Stimulation durch den Antikörper 103/51 nicht durch andere Rezeptoren als den CFR-1 vermittelt wurde, eine MTT-Stimulationsuntersuchung mit der Zellinie 23132 vorgenommen und mit der CFR-1 negativen Lungenkarzinomzellinien Colo-699 und EPLC-272H verglichen. Während die Zellinie 23132, wie oben beschrieben, stimuliert wird, zeigten die beiden Lungenkarzinomzellinien keine Stimulation durch den Antikörper 103/51 (Fig. 7 d), was die Resultate der Immunhistochemie bestätigt.

**Tab. 1**

| a) Tumor tissues | | | b) Normal tissues | | |
|---|---|---|---|---|---|
| Tissue | Carcinoma-type | Antibody-Staining | Tissue | Cell type | Antibody-Staining |
| Esophagus | Squamous | + | sallvary gland | Glandular | - |
| Stomach | Adeno (diffuse) | ++ | Stomach (non Inflammated) | Glandular | - |
| Stomach | Adeno (intestinal) | + | Stomach (*H. pylori* Infected) | Glandular | + |
| Colon | Adeno | + | Stomach (high grade dysplasia) | Glandular | ++ |
| Rectum | Adeno | + | Duodenum | Glandular | - |
| Liver | Adeno (HCC) | ++ | Colon | Epithelial | - |
| Gallbladder | Adeno | + | Rectum | Glandular | - |
| Pancreas | Adeno (ductal) | + | Pancreas | Glandular | - |
| Papilla of Vater | Adeno | + | Liver | Glandular | - |
| Lung | Large cell anaplastic | - | Gallbladder | Glandular | - |
| Lung | Small cell | - | Oral mucosa | Squamus epithelium | - |
| Lung | Adeno | ++ | Anal mucosa | Squamous epithelium | - |
| Bronchus | Squamous epithelium | + | Skin | Keralinocyte glandular | - |
| Mamma | Invasive (ductal) | + | Mamma | Glandular | - |
| Mamma | Invasive (lobular) | + | Larynx | Epithelial | - |
| | | | Bronchus | Epithelial | - |
| | | | Lung | Glandular, alveolar | - |
| | | | Thyroid gland | Glandular | - |
| | | | Adenohypophysis | Glandular - | |
| | | | Adrenal gland | Glandular | ++² |
| | | | Testis | Glandular | - |
| | | | Ovar | Glandular | - |
| | | | Prostate | Glandular | - |
| | | | Urothelium | Epithelial | - |
| | | | Kidney | Epithelial | ++¹ |
| | | | Thymus | Lymphalic | - |
| | | | Spieen | Lymphalic | - |
| | | | Lymph node | Lymphatic | - |
| | | | Cerebral cortex | Neural | - |
| | | | Peripheric neural ganglia | Neural | - |

<110> Prof. Dr. Müller-Hermelink, Hans Konrad
   Prof. Dr. Vollmers, Heinz
   Dr. Hensel, Frank
<120> Rezeptor, dessen Verwendung und Mausantikörper
<141> 2002-03-09
<211> 288 bp
   <212> DNA
   <213> Mus Musculus
   <220> Sequenz der variablen Region der schweren Kette (V_{H}) des Antikörpers NM5 8-49/69
   <221> V-Region
   <222> (1)......(288)
<400> 
tcc tgc aag gct tct ggc tac acc ttc act gac tac tat ata aac tgg gtg aag cag agg 60
Ser Cys Lys Ala Ser Gly Tyr Thr Phe Thr Asp Tyr Tyr Ile Asn Trp Val Lys Gln Arg
1               5                   10                  15                  20

act gga cag ggc ctt gag tgg att gga gag att tat cct gga agt ggt aat act tac tac 120
Thr Gly Gln Gly Leu Glu Trp Ile Gly Glu Ile Tyr Pro Gly Ser Gly Asn Thr Tyr Tyr
                25                  30                  35                  40

aat gag aag ttc aag ggc aag gcc aca ctg act gca gac aaa tcc tcc agc aca gcc tac 180
Asn Glu Lys Phe Lys Gly Lys Ala Thr Leu Thr Ala Asp Lys Ser Ser Ser Thr Ala Tyr
                45                  50                  55                  60

atg cag ctc agc agc ctg aca tct gag gac tct gca gtc tat ttc tgt gca aga tcg gga 240
Met Gln Leu Ser Ser Leu Thr Ser Glu Asp Ser Ala Val Tyr Phe Cys Ala Arg Ser Gly
50 55 60 65

tta cga ccc tat gct atg gac tac tgg ggt caa gga acc tca gtc acc 288
Leu Arg Pro Tyr Ala Met Asp Tyr Trp Gly Gln Gly Thr Ser Val Thr
70 75 80
<110> Prof. Dr. Müller-Hermelink, Hans Konrad
   Prof. Dr. Vollmers, Heinz
   Dr. Hensel, Frank
<120> Rezeptor, dessen Verwendung und Mausantikörper
<141> 2002-03-09
<211> 315 bp
   <212> DNA
   <213> Mus Musculus
   <220> Sequenz der variablen Region der leichten Kette (V_{L}) des Antikörpers NM58-49/69
   <221> V-Region
   <222> (1).....(315)
<400> 
cca ctc tcc ctg cct gtc agt ctt gga gat caa gcc tcc atc tct tgc aga tct agt cag 60
Pro Leu Ser Leu Pro Val Ser Leu Gly Asp Gln Ala Ser Ile Ser Cys Arg Ser Ser Gln
1               5                   10                  15                  20

agc att gta cat agt aat gga aac acc tat tta gaa tgg tac ctg cag aaa cca ggc cag 120
Ser Ile Val His Ser Asn Gly Asn Thr Tyr Leu Glu Trp Tyr Leu Gln Lys Pro Gly Gln
                25                  30                  35                  40

tct cca aag ctc ctg atc tac aaa gtt tcc aac cga ttt tct ggg gtc cca gac agg ttc 180
Ser Pro Lys Leu Leu Ile Tyr Lys Val Ser Asn Arg Phe Ser Gly Val Pro Asp Arg Phe
                45                  50                  55                  60

agt ggc agt gga tca ggg aca gat ttc aca ctc aag atc agc aga gtg gag gct gag gat 240
Ser Gly Ser Gly Ser Gly Thr Asp Phe Thr Leu Lys Ile Ser Arg Val Glu Ala Glu Asp
                65                  70                  75                  80

ctg gga gtt tat tac tgc ttt caa ggt tca cat gtt ccg tac acg ttc gga ggg ggg acc 300
Leu Gly Val Tyr Tyr Cys Phe Gln Gly Ser His Val Pro Tyr Thr Phe Gly Gly Gly Thr
                85                  90                  95                  100

aag ctg gaa ata aaa 315
Lys Leu Glu Ile Lys
                105
<110> Prof. Dr. Müller-Hermelink, Hans Konrad
   Prof. Dr. Vollmers, Heinz
   Dr. Hensel, Frank
<120> Rezeptor, dessen Verwendung und Mausantikörper
<141> 2002-03-09
   <211> 3114
<212> DNA
   <213> Homo sapiens
   <220> Cysteine-rich FGF Rezeptor der Magenkarzinonozellinie 23132
   <221> CDS
   <222> (450).....(3563)
<400>

450 GAT GTG AGG GAG CCT GAA AAT GAA ATT TCT TCA GAC TGC AAT CAT TTG TTG TGG AAT TAT 509
Asp Val Arg Glu Pro Glu Asn Glu Ile Ser Ser Asp Cys Asn His Leu Leu Trp Asn Tyr
143 145 150 155 160

AAG CTG AAC CTA ACT ACA GAT CCC AAA TTT GAA TCT GTG GCC AGA GAG GTT TGC AAA TCT 569
Lys Leu Asn Leu Thr Thr Asp Pro Lys Phe Glu Ser Val Ala Arg Glu Val Cys Lys Ser
165 170 175 180

ACT ATA ACA GAG ATT GAA GAA TGT GCT GAT GAA CCG GTT GGA AAA GGT TAC ATG GTT TCC 629
Thr Ile Thr Glu Ile Glu Glu Cys Ala Asp Glu Pro Val Gly Lys Gly Tyr Met Val Ser
185 190 195 200

TGC TTG GTG GAT CAC CGA GGC AAC ATC ACT GAG TAT CAG TGT CAC CAG TAC ATT ACC AAG 689
Cys Leu Val Asp His Arg Gly Asn Ile Thr Glu Tyr Gln Cys His Gln Tyr Ile Thr Lys
205 210 215 220

ATG ACG GCC ATC ATT TTT AGT GAT TAC CGT TTA ATC TGT GGC TTC ATG GAT GAC TGC AAA 749
Met Thr Ala Ile Ile Phe Ser Asp Tyr Arg Leu Ile Cys Gly Phe Met Asp Asp Cys Lys
225 230 235 240

AAT GAC ATC AAC ATT CTG AAA TGT GGC AGT ATT CGG CTT GGA GAA AAG GAT GCA CAT TCA 809
Asn Asp Ile Asn Ile Leu Lys Cys Gly Ser Ile Arg Leu Gly Glu Lys Asp Ala His Ser
245 250 255 260

CAA GGT GAG GTG GTA TCA TGC TTG GAG AAA GGC CTG GTG AAA GAA GCA GAA GAA AGA GAA 869
Gln Gly Glu Val Val Ser Cys Leu Glu Lys Gly Leu Val Lys Glu Ala Glu Glu Arg Glu
265 270 275 280

CCC AAG ATT CAA GTT TCT GAA CTC TGC AAG AAA GCC ATT CTC CGG GTG GCT GAG CTG TCA 929
Pro Lys Ile Gln Val Ser Glu Leu Cys Lys Lys Ala Ile Leu Arg Val Ala Glu Leu Ser
285 290 295 300

TCG GAT GAC TTT CAC TTA GAC CGG CAT TTA TAT TTT GCT TGC CGA GAT GAT CGG GAG CGT 989
Ser Asp Asp Phe His Leu Asp Arg His Leu Tyr Phe Ala Cys Arg Asp Asp Arg Glu Arg
305 310 315 320

TTT TGT GAA AAT ACA CAA GCT GGT GAG GGC AGA GTG TAT AAG TGC CTC TTT AAC CAT AAA 1049
Phe Cys Glu Asn Thr Gln Ala Gly Glu Gly Arg Val Tyr Lys Cys Leu Phe Asn His Lys
325 330 335 340

TTT GAA GAA TCC ATG AGT GAA AAG TGT CGA GAA GCA CTT ACA ACC CGC CAA AAG CTG ATT 1109
Phe Glu Glu Ser Met Ser Glu Lys Cys Arg Glu Ala Leu Thr Thr Arg Gln Lys Leu Ile
345 350 355 360

GCC CAG GAT TAT AAA GTC AGT TAT TCA TTG GCC AAA TCC TGT AAA AGT GAC TTG AAG AAA 1169
Ala Gln Asp Tyr Lys Val Ser Tyr Ser Leu Ala Lys Ser Cys Lys Ser Asp Leu Lys Lys
365 370 375 380

TAC CGG TGC AAT GTG GAA AAC CTT CCG CGA TCG CGT GAA GCC AGG CTC TCC TAC TTG TTA 1229
Tyr Arg Cys Asn Val Glu Asn Leu Pro Arg Ser Arg Glu Ala Arg Leu Ser Tyr Leu Leu
385 390 395 400

ATG TGC CTG GAG TCA GCT GTA CAC AGA GGG CGA CAA GTC AGC AGT GAG TGC CAG GGG GAG 1289
Met Cys Leu Glu Ser Ala Val His Arg Gly Arg Gln Val Ser Ser Glu Cys Gln Gly Glu
405 410 415 420

ATG CTG GAT TAC CGA CGC ATG TTG ATG GAA GAC TTT TCT CTG AGC CCT GAG ATC ATC CTA 1349
Met Leu Asp Tyr Arg Arg Met Leu Met Glu Asp Phe Ser Leu Ser Pro Glu Ile Ile Leu
425 430 435 440

AGC TGT CGG GGG GAG ATT GAA CAC CAT TGT TCC GGA TTA CAT CGA AAA GGG CGG ACC CTA 1409
Ser Cys Arg Gly Glu Ile Glu His His Cys Ser Gly Leu His Arg Lys Gly Arg Thr Leu
445 450 455 460

CAC TGT CTG ATG AAA GTA GTT CGA GGG GAG AAG GGG AAC CTT GGA ATG AAC TGC CAG CAG 1469
His Cys Leu Met Lys Val Val Arg Gly Glu Lys Gly Asn Leu Gly Met Asn Cys Gln Gln
465 470 475 480

GCG CTT CAA ACA CTG ATT CAG GAG ACT GAC CCT GGT GCA GAT TAC CGC ATT GAT CGA GCT 1529
Ala Leu Gln Thr Leu Ile Gln Glu Thr Asp Pro Gly Ala Asp Tyr Arg Ile Asp Arg Ala
485 490 495 500

TTG AAT GAA GCT TGT GAA TCT GTA ATC CAG ACA GCC TGC AAA CAT ATA AGA TCT GGA GAC 1589
Leu Asn Glu Ala Cys Glu Ser Val Ile Gln Thr Ala Cys Lys His Ile Arg Ser Gly Asp
505 510 515 520

CCA ATG ATC TTG TCG TGC CTG ATG GAA CAT TTA TAC ACA GAG AAG ATG GTA GAA GAC TGT 1649
Pro Met Ile Leu Ser Cys Leu Met Glu His Leu Tyr Thr Glu Lys Met Val Glu Asp Cys
525 530 535 540

GAA CAC CGT CTC TTA GAG CTG CAG TAT TTC ATC TCC CGG GAT TGG AAG CTG GAC CCT GTC 1709
Glu His Arg Leu Leu Glu Leu Gln Tyr Phe Ile Ser Arg Asp Trp Lys Leu Asp Pro Val
545 550 555 560

CTG TAC CGC AAG TGC CAG GGA GAC GCT TCT CGT CTT TGC CAC ACC CAC GGT TGG AAT GAG 1769
Leu Tyr Arg Lys Cys Gln Gly Asp Ala Ser Arg Leu Cys His Thr His Gly Trp Asn Glu
565 570 575 580

ACC AGC GAA TTT ATG CCT CAG GGA GCT GTG TTC TCT TGT TTA TAC AGA CAC GCC TAC CGC 1829
Thr Ser Glu Phe Met Pro Gln Gly Ala Val Phe Ser Cys Leu Tyr Arg His Ala Tyr Arg
585 590 595 600

ACT GAG GAA CAG GGA AGG AGG CTC TCA CGG GAG TGC CGA GCT GAA GTC CAA AGG ATC CTA 1889
Thr Glu Glu Gln Gly Arg Arg Leu Ser Arg Glu Cys Arg Ala Glu Val Gln Arg Ile Leu
605 610 615 620

Cys Leu Lys Gln Asn Lys Asn Ser Glu Leu Met Asp Pro Lys Cys Lys Gln Met Ile Thr
905 910 915 920

AAG CGC CAG ATC ACC CAG AAC ACA GAT TAC CGC TTA AAC CCC ATG TTA AGA AAA GCC TGT 2849
Lys Arg Gln Ile Thr Gln Asn Thr Asp Tyr Arg Leu Asn Pro Met Leu Arg Lys Ala Cys
925 930 935 940

AAA GCT GAC ATT CCT AAA TTC TGT CAC GGT ATC CTG ACT AAG GCC AAG GAT GAT TCA GAA 2909
Lys Ala Asp Ile Pro Lys Phe Cys His Gly Ile Leu Thr Lys Ala Lys Asp Asp Ser Glu
945 950 955 960

TTA GAA GGA CAA GTC ATC TCT TGC CTG AAG CTG AGA TAT GCT GAC CAG CGC CTG TCT TCA 2969
Leu Glu Gly Gln Val Ile Ser Cys Leu Lys Leu Arg Tyr Ala Asp Gln Arg Leu Ser Ser
965 970 975 980

GAC TGT GAA GAC CAG ATC CGA ATC ATT ATC CAG GAG TCC GCC CTG GAC TAC CGC CTG GAT 3029
Asp Cys Glu Asp Gln Ile Arg Ile Ile Ile Gln Glu Ser Ala Leu Asp Tyr Arg Leu Asp
985 990 995 1000

CCT CAG CTC CAG CTG CAC TGC TCA GAC GAG ATC TCC AGT CTA TGT GCT GAA GAA GCA GCA 3089
Pro Gln Leu Gln Leu His Cys Ser Asp Glu Ile Ser Ser Leu Cys Ala Glu Glu Ala Ala
1005 1010 1015 1020

GCC CAA GAG CAG ACA GGT CAG GTG GAG GAG TGC CTC AAG GTC AAC CTG CTC AAG ATC AAA 3149
Ala Gln Glu Gln Thr Gly Gln Val Glu Glu Cys Leu Lys Val Asn Leu Leu Lys Ile Lys
1025 1030 1035 1040

ACA GAA TTG TGT AAA AAG GAA GTG CTA AAC ATG CTG AAG GAA AGC AAA GCA GAC ATC TTT 3209
Thr Glu Leu Cys Lys Lys Glu Val Leu Asn Met Leu Lys Glu Ser Lys Ala Asp Ile Phe
1045 1050 1055 1060

GTT GAC CCG GTA CTT CAT ACT GCT TGT GCC CTG GAC ATT AAA CAC CAC TGC GCA GCC ATC 3269
Val Asp Pro Val Leu His Thr Ala Cys Ala Leu Asp Ile Lys His His Cys Ala Ala Ile
1065 1070 1075 1080

ACC CCT GGC CGC GGG CGT CAA ATG TCC TGT CTC ATG GAA GCA CTG GAG GAT AAG CGG GTG 3329
Thr Pro Gly Arg Gly Arg Gln Met Ser Cys Leu Met Glu Ala Leu Glu Asp Lys Arg Val
1085 1090 1095 1100

AGG TTA CAG CCC GAG TGC AAA AAG CGC CTC AAT GAC CGG ATT GAG ATG TGG AGT TAC GCA 3389
Arg Leu Gln Pro Glu Cys Lys Lys Arg Leu Asn Asp Arg Ile Glu Met Trp Ser Tyr Ala
1105 1110 1115 1120

GCA AAG GTG GCC CCA GCA GAT GGC TTC TCT GAT CTT GCC ATG CAA GTA ATG ACG TCT CCA 3449
Ala Lys Val Ala Pro Ala Asp Gly Phe Ser Asp Leu Ala Met Gln Val Met Thr Ser Pro
1125 1130 1135 1140

TCT AAG AAC TAC ATT CTC TCT GTG ATC AGT GGG AGC ATC TGT ATA TTG TTC CTG ATT GGC 3509
Ser Lys Asn Tyr Ile Leu Ser Val Ile Ser Gly Ser Ile Cys Ile Leu Phe Leu Ile Gly
1145 1150 1155 1160

CTG ATG TGT GGA CGG ATC ACC AAG CGA GTG ACA CGA GAG CTC AAG GAC AGG TAG 3563
Leu Met Cys Gly Arg Ile Thr Lys Arg Val Thr Arg Glu Leu LysA Asp Arg ***
1165 1170 1175 1179

## Patentansprüche

1. Antikörper mit einer variablen Region der schweren Kette mit einer Struktur gemäß Anlage A und einer variablen Region der leichten Kette mit einer Struktur gemäß Anlage B.

## Claims

1. Antibody with a heavy chain variable region having a structure according to Exhibit A and a light chain variable region having a structure according to Exhibit B.

## Revendications

1. Anticorps avec une région variable de la chaîne lourde avec une structure selon l'annexe A et une région variable de la chaîne légère avec une structure selon l'annexe B.
